# EUROPEAN PATENT APPLICATION

(11) **EP 1 952 811 A1**
(43) Date of publication of application: **06.08.2008**
(21) Application number: 06791205.5
(22) Date of filing: 08.10.2006
(51) Int. Cl.: A61K 31/352, A61P 35/02

(54) **THE USE OF ERIOCALYXIN B IN THE MANUFACTURE OF MEDICAMENTS FOR TREATING LEUKEMIA**

(30) Priority: 07.11.2005 CN 200510110089
(71) Applicant: Rui Jin Hospital Affiliated to Shanghai Second Medical University, Shanghai 200025 (CN); Kunming Institute of Botany, The Chinese Academy of Scienes, Kunming, Yunnan 650204 (CN)
(72) Inventor: CHEN, Sai-Juan, Shanghai 200025 (CN); CHEN, Zhu, Shanghai 200025 (CN); WANG, Lan, Shanghai 200025 (CN); ZHAO, Wei-Li, Shanghai 200025 (CN); SUN, Han-Dong, Yunnan 650204 (CN)
(74) Representative: Jeannet, Olivier
(86) International application number: PCT/CN2006/002623
(87) International publication number: WO 2007/051390

(57) **Abstract**

The use of eriocalyxin B in the manufacture of medicaments for treating leukemia, wherein the leukemia include leukemia containing AML1-ETO fusion protein, acute promyelocytic leukemia, acute myelocytic leukemia and lymphocytic leukemia. Eriocalyxin B increases the level of peroxidate and influences the phosphorylation and degradation of IκBα to prevent NF-κB from getting into the nucleus in order to inhibition NF-κB pathway. Eriocalyxin B can decrease the phosphorylation of ERI1/2 and inhibit MAPK pathway. Eriocalyxin B can extend the life span of mice and decrease tumor volume in mice model of C57 leukemia and tumor transplantation.

## Description

### TECHNICAL FIELD OF THE INVENTION

The present invention relates to the application of eriocalyxin B, and more particularly to the application of eriocalyxin B in manufacture of medicaments.

### BACKGROUND OF THE INVENTION

Malignant tumors are serious diseases that endanger human health and lives. At the present time, China's number of annual malignancy incidences is about 1.6 million, and malignant tumor has leapt to the first place in the cause of death for urban residents. Leukemia is a general name for malignant tumors of bone marrow cells, lymphocytes, and other hematopoietic cells. Acute myeloid leukemia (AML) is a group of hematopoietic system malignancies originated from the abnormal proliferation, differentiation, and survival of the myeloid progenitor cells. Distinctive chromosomal translocations often appear in AML cases and play a key role in leukemia incidences. In AML cases, t(8;21)(q22;q22) represents the most commonly seen chromosomal translocations, and the chromosomal translocation generated AML1-ETO fusion protein. AML1-ETO blocks myeloid cell differentiation and apoptosis, and plays a key role in disease pathogenesis. Clinically, patients with t(8;21) AML receive chemotherapy and 40% - 50% of them still have relapses afterwards, in that, the patients also become drug resistant. Moreover, the high-dosage chemotherapy is unsuitable for elderly patients. Malignant tumors greatly endanger human lives and health. Therefore, it is of great significance to develop an alternative drug or a substitute agent which is able to directly target against malignant tumors in reducing mortality, providing clinical guidance, and also improving the quality of life of tumor patients.

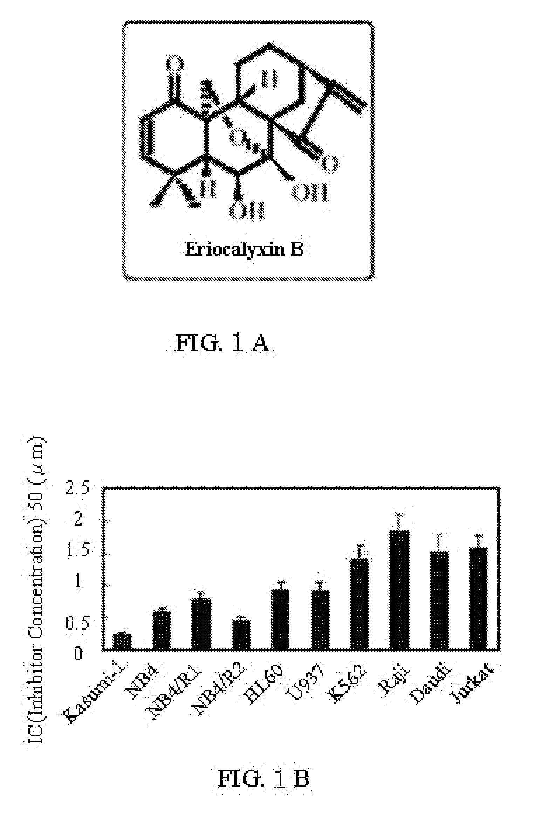

In the past 30 years, natural products in the treatment of cancer have shown promising results. Natural products can provide many leading precursor chemical compounds, which can be used to design more biologically active templates for new drugs. Paclitaxel (trade name Taxol) is a natural product from the bark of Pacific yew Taxus brevifolia. Paclitaxel is effective for drug resistant cancers of breast and ovarian and more than 100 species of syntheses of Paclitaxel have been used by clinical applications. Inorganic arsenic compounds, for example, initially originated from the arsenic trioxide, are very effective in the treatment of acute promyelocytic leukemia, the M3 subtype of AML characterized by t(15;17) and resultant PML-RAR fusion gene. Oridonin, a diterpene compound extracted from the medicinal plants Isodon rubescens, exhibits a strong anti-tumor activity. Eriocalyxin B, abbreviated as EriB (see FIG. 1A), is an ent-kauran-diterpene compound isolated from a medicinal plant Isodon eriocalyxlsodon eriocalyx var. laxiflora (Shu Hua Eriocalyxin Isodon), which is found in China and uniquely distributed in the southwest region of China. The two afore-said medicinal plants are used in the civil society as anti-inflammatory and antibacterial drugs. EriB displays cytotoxic effect in a variety of cancer cell lines. EriB can significantly inhibit HL60, A549, MKN-28, HCT and CA cell growth through responses with biological macromolecules, such as sulfthydryl, as multi-targets to exercise the EriB activities.

### SUMMARY OF THE INVENTION

The objectives of the present invention are: (1) Providing an application of eriocalyxin B (EriB) in manufacture of medicaments for treating acute leukemia; and (2) Providing the application of eriocalyxin B in manufacture of medicaments for treating Burkitt's lymphoma.

The objectives of the present invention are achieved by the following technical methods:

(I) Reagents : EriB (provided by professor Sun Han Dong of Kunming Institute of Botany, Chinese Academy of Sciences) is dissolved in DMSO as a 100mM concentration stock solution at -20°C. MTT, propidium iodide (PI), dichlorofluorescein diacetate (DCFH-DA), tumor necrosis factor alpha (TNF-α), dithiothreitol (disulfide-sugar alcohol), protease inhibitors, and Hochest 33258, were purchased from Sigma company. Z-DEVD-fmk was purchased from BD company. Anti-PARP, caspase-3, Bcl2, Bcl-XL, Bax, P65, Lamin B, carboxyl-terminal IκBα, amino-terminal IκBα, ERK1 / 2, phosphorylated form of ERK1 / 2, c-Jun, phosphorylated form of c-Jun and ETO antibodies, peroxidase-coupled goat anti-mouse IgG, goat anti-rabbit IgG, and mouse anti-goat IgG, were purchased from the Santa Cruz company. Anti-β-actin and c-Fos antibody were purchased from Abcam company. Anti-cleaved caspase-3, IκBα and chemiluminescence detection kits were purchased from Cell Signaling company. Donkey anti-mouse fluorescent-labeled IgG was purchased from Molecular Probes company.

(II) Cell culture, cell survival and cell morphology: the present invention utilized the following human cell lines: AML leukemia with t(8;21) chromosomal translocation Kasumi-1 cell lines, which is also known as acute myeloid leukemia comprising AML1-ETO fusion gene; acute promyelocytic leukemia NB4, NB4/R1 and NB4/R2 cell lines, and acute myeloid leukemia U937 cell lines; Burkitt's lymphoma (Raji and Daudi) cell lines; T-cell acute lymphoblastic leukemia (Jurkat) cell lines. The present invention studied by collecting primary leukemia cells from five AML patients with t(8;21). The diagnoses of patients were established on the bases of morphological examination, cytogenetics study detection of t(8;21) chromosomal translocation, and RT-PCR detection of AML1-ETO fusion protein. Leukemia cells were prepared in the following conditions: separated with Lymphocyte Separation Medium, further isolated and cultured in RPMI-1640 medium (Gibco / BRL), in addition, added 10% FBS (PAA) in 5%CO₂-95% air and saturated humidity, and at 37°C. In each experiment, cell inoculation density is 2×10⁵/ml. The survival rate of cells was accessed using trypan blue dye-exclusion experiments. Cell observations were done by using the Wright's staining method.

(III) MTT experiment: cells were treated with EriB at concentrations of 0.1, 0.25, 0.5, 1, 2, 4, 6, 8 and 10µM in a 96-hole plate. After 72 hours, 0.1 mg MTT was added to each hole, incubated at 37°C for four hours later, absorbance values were measured at 570nm by spectrophotometer.

(IV) Flow cytometric for annexin-V, cell cycle, mitochondrial transmembrane potential and reactive oxygen species (ROS) generation: Cell apoptosis was analyzed using an ApoAlert Annexin V-FITC Apoptosis kit (BD). In the process of analyzing the cell cycle, the cells were fixed overnight in methanol at -20°C, followed by treatment of Tris-HCI buffer (pH 7.4) comprising 1% RNase, 50µg/ml PI staining. In the detection of mitochondrial transmembrane potential, the cells were washed with PBS, incubated at 37°C and with 10mg/ml Rh123 for 30 minutes, and then staining with 50µg/ml PI. DCFH-DA was used to detect ROS levels, thus the cells were washed with PBS, and incubated at 37°C and with 20mM DCFH-DA for 30 minutes. Fluorescence intensity was measured by flow cytometry.

(V) Transmission electron microscope: cells were fixed in 2% glutaraldehyde at 4°C overnight and centrifuged at 3000×*g* for 15 minutes, then washed with 0.1M cacodylate buffer, and fixed in 1% osmium tetroxide for one hour at 4°C, and after dehydration, embedded with Epon 812. The samples were prepared using a thin slicing machine, and after staining, observations were done on transmission electron microscope.

(VI) Immunofluorescence: the cells were fixed in methanol for five minutes at -20°C, after being washed with PBS, treated by 0.5% Triton-X-permeability, blocked with 1% BSA, and incubated with anti-P65 antibody at room temperature for one hour; after being washed again with PBS and incubated with the fluorescent-label IgG at room temperature for 30 minutes. The cells were incubated with Hochest 33258 for five minutes later, and observations were proceeded under the fluorescent microscope.

(VII) Western Blot analysis: lysing 5x10⁶ cells with 200 µl Laemmli lysis buffer (0.5M Tris-HCI, pH 6.8, 2mM EDTA, 10% glycerol, 2% SDS and 5% β-mercaptoethanol). Protein lysate (20µg) was used on 10% polyacrylamide gel electrophoresis, and then transferred to nitrocellulose membranes. Nitrocellulose membranes were blocked with TBS/0.05% Tween 20 5% skim (nonfat) milk, and afterwards, incubated with primary antibody for two hours at room temperature, and incubated with horseradish peroxidase labeled IgG for one hour. The Immunocomplexes were detected with chemiluminescence horseradish peroxidase detection kit. The signal intensities of the respective bands were analyzed with Quantity One version 4.1.1 software.

(VIII) Nucleoprotein and pulp protein Separation: 1×10⁷ cells and 400µl of 0.2% Nonidet P-40 and the protease inhibitor lysis buffer (10mM HEPES, 10mM KCI, 1.5mM MgCl₂, 0.5mM DTT, pH 7.9) were incubated on ice for one minute. Centrifugation at 2500×*g* was performed for one minute, and afterwards, supernatants were collected as plasma protein. Precipitation was washed with non-NP-40 lysis buffer, then resuspended in extraction buffer (20mM HEPES, pH 7.9, 420mM NaCl, 0.5mM DTT, 0.2mM EDTA and 25% glycerol), and incubated on ice for 20 minutes. Centrifugation at 12000×*g* was performed for 10 minutes, and afterwards, the supernatant is collected as the nucleoprotein.

(IX) Semi-quantitative reverse transcription-PCR (RT-PCR): the total RNA was extracted using Trizol (Invitrogen). Random hexamers and Superscript II (Invitrogen) ART were used for reverse transcribed into cDNA. The present invention utilized the following primers:
GAPDH,5'-TCACCAGGGCTGCTTTTA-3' and 5'-AAGGTCATCCCTGAGCTGAA-3';
Bcl-2,5'-GCAGGCATGTTGACTTCACTT-3' and 5'-GGAGGATTGTGGCCTTCTTTG-3':
Bcl-XL5'-CATGGCAGCAGTAAAGCAAGC-3' and 5'-TGCGATCCGACTCACCAATAC-3'
Bax,5'TTCTGACGGCAACTTCAACTGGG-3'5'-TTCTTCCAGATGGTGAGCGAGG-3'. Cycle parameters: 94°C for 5 minutes; 94 °C for 1 minute, 58 °C for 1 minute, 72°C for 1 minute, a total of 28 cycles; 72 °C for 5 minutes.

(X) Luciferase report assay: Kasumi-1 cells were resuspended in cultured medium, added the gene vector [10µg, NF-κB-mediated luciferase reporter plasmid or pAP1-luc (Clontech), and 2µg of β-galactosidase enzyme expression plasmid for correcting cell transfection efficiency of the CMV-mediated. Cell transfection conditions were: 1000V, 25µF, and the instrument came from the Bio-Rad Laboratories, Inc. Transfections after 36 hours, the cells were treated with EriB and/or TNFα, and then the cells were lysed. Luciferase activity was measured by lumat LB 9507 tube luminometer (EG&G Berthold). Cell lysates were mixed with 2 × β-half-galactosidase reaction reagents, after incubated for one hour at 37°C, the termination of reaction by adding 1 M Na₂CO₃. The β-galactosidase enzyme absorbance values were measured at 420nm by spectrophotometer.

(XI) Gel electrophoresis shift assay: nucleoprotein extracts of Kasumi-1 cells were quantitatively determined by using Coomassie plus reagent (Pierce). Nucleoprotein (10µg) and ³²P marked double-stranded NF-κB (5'-AGTTGAGGGGACTTTCCCAGGC-3') were sequentially incubated, and then lysed using 4% of non-denaturing PAGE gel; the marked bands were detected by using autoradiography.

(XII) Leukemia murine model: female C57 mice and nude mice (5-6 weeks) were bred in special pathogen-free environment. C57 mice received 400 cGy from a high-energy linear accelerator at the dose rate of 100 cGy per minute. After 24 hours, the C57 mice were injected with 3×10⁶ Mig/Etr cells expressing carbon-terminal truncated AML1-ETO of 0.2ml PBS via tail vein intravenous injection. Treatments (12 mice in each group) were started five days after injection of leukemia cells. The control group and the EriB treated group, all respectively receive EriB (1.25, or 2.5mg/kg, intraperitoneal injection for two weeks) and diluents (1% Pluronic F68).

(XIII) Statistical analysis: the results were expressed by using the average value and the standard deviation of three independent experiments, using t-test to compare the differences. P-values <0.05 were considered statistically significant. All statistical analyses were evaluated using SAS 8.2 software.

Diterpenoids show significant cytotoxicity against solid tumor cells and hematological malignant cells. Through the present invention studies, we found that EriB, a diterpene compound isolated from Isodon eriocalyx var. laxiflora, possesses an antiproliferation effect on leukemia/lymphoma cells, especially on t(8;21) leukemia cells. EriB induces apoptosis of Kasumi-1 cells and fresh leukemic cells from patients, which suggests that EriB is effective in treating t(8;21) leukemia. EriB is able to directly target AML1-ETO fusion protein, mainly through activation of caspases. These have been demonstrated by morphological characteristics, and proved by the results of sub-G1 cells and annexin V⁺/PI⁻cell assays. Moreover, EriB causes collapse of mitochondrial transmembrane potential in Kasumi-1 cells. It has been reported that Oridonin, a diterpenoid isolated from Rabdosia rubescens, downregulates the Bcl-2 and induces apoptosis of NB4 and K562 cells. In the present invention studies, EriB has been shown to be able to significantly reduce Bcl-2 and Bcl-X_{L} at mRNA and protein levels, which further suggests that the EriB-induced apoptosis involve intrinsic apoptosis pathway.

Constitutive activation of NF-κB is observed in AML cells, and NF-κB regulates expression of many genes that are important for the apoptosis and survival of leukemia cells, including Bcl-2 and Bcl-X_{L}. NF-κB transcription factors can form homo-dimers and hetero-dimers with many factors including TNF-α. Degradation of IκBα leads to translocation of NF-κB from cytoplasm into the nucleus and initiating transcription activation of targeted gene. Recent studies have shown that oridonin treatment is associated with blockage of NF-κB signaling pathways. In one aspect, oridonin directly interferes with the DNA-binding activity of NF-κB to its response DNA sequence; in another aspect, oridonin has an additional impact on NF-κB nuclear translocation by affecting IκBα phosphorylation and degradation. In the present invention studies, we found that two different signaling pathways were involved in EriB-induced inhibition of NF-κB activation. EriB inhibits the intrinsic NF-κB activation by directly interrupting the DNA-binding activity of NF-κB without the change of nuclear translocation. In the presence of TNF-α, EriB prevented TNF-α-induced NF-κB activation through blocking IκBα phosphorylation and degradation. Therefore, EriB has the ability to negatively regulate two important aspects of NF-κB activation process. Given the expression of NF-κB in AML, but not normal primitive cells, inhibition of NF-κB might induce leukemia cell apoptosis.

NF-κB is a redox-sensitive transcription factor. NF-κB and its target genes integration in the nucleus require a reductive environment. In NF-κB and the DNA-binding region, the redox-sensitive homocysteine may be oxidized, thus making NF-κB unable to bind with the targeted genes. In the present invention studies, we found that in Kasumi-1 cells treated with EriB, EriB can increase ROS at an early stage; then NF-κB was inhibited and the cells committed apoptosis. Antioxidants DTT can be completely inhibited by the EriB induction in a series of changes. The results are shown in the process of EriB induced NF-κB inhibition and reduction, that is, the balance of oxidation is very important. On the other hand, EriB is recognized to be able to combine with sulfhydryl of protein. EriB-induced NF-κB inhibition may also be due to EriB and NF-κB DNA-binding region of homocysteine on the sulfhydryl combination. When sulfhydryl of homocysteine formed disulfide bonds, homocysteine is oxidized, which will make NF-κB not able to be combined with the targeted genes. DTT can reduce the formation of disulfide bonds, making cysteine residues to reinstate the original state and restore NF-κB activity.

Caspase-3 activation is essential for leukemia cell apoptosis. In addition to playing a role in apoptosis, caspase-3 can also cleave IκBα at ser32, leading to loss of IκBα ubiquitination site and generating ΔIκBα, the non-degradable form of IκBα. ΔIκBα acts as a super-inhibitor in its process of integrating with the NF-κB dimer and decreases the NF-κB activity through the gathering of NF-κB in the cytoplasm. EriB can activate caspase-3, followed by cleaving PARP. In the present invention studies, we detected a 34kDa size of IκBα cleaved product (ΔIκBα) corresponding to this phenomenon of caspase-3 increased activity and the reduction of NF-κB protein level in the nucleus. Caspase-3 activation seems to be related to EriB induced degradation of AML1-ETO, but only partially reducing EriB induced apoptosis, and this prompted that EriB induced apoptosis process still exists a caspase-3-independent mechanism.

Raf/MEK/ERK cascade generally promotes cell survival, particularly in malignant hematopoietic cells. Interference of ERK1 / 2 may reduce the threshold of EriB induced apoptosis. AP-1 activation can promote cell death, in mammalian cells in the main AP-1 complexes members are c-Jun and c-Fos. Some studies showed that when cells are faced with DNA damage, c-Jun can induce CD95-L to pro-apoptotic regulation, and the c-Fos has both pro-apoptotic and anti-apoptotic function, depending on the cell type and extra-cellular stimuli. Oridonin is a diterpene compound isolated from Isodon rubescen, and it can increase the apoptotic rate of the L929 cells through the activation of ERK1 / 2 dependent MAPK pathway. In the present invention studies, we proved that in Kasumi-1 cells, EriB rapidly inhibited ERK1 / 2 phosphorylation, resulting in activation of transcription factor AP-1. This shows that EriB can induce pro-survival signaling regulator (for example: ERK1 / 2) as well as pro-apoptosis regulator (for example: AP-1). In the process of EriB induced apoptosis, this may be caspase-3 independent mechanism.

In summary, the current studies prove that EriB induces t(8;21) apoptosis by inhibiting NF-κB and MAPK signaling pathway. EriB is a potent apoptosis inducer for t(8;21) leukemia cells and a potential therapeutic medicament. The present invention discloses an application of EriB in the manufacture of medicaments having the advantages of: (1) The present invention has explored a new medical application of EriB, which has opened up a new application field. (2) The present invention shows that EriB is safe and non-toxic, has strong pharmacological effects, and indicates a very good prospect for medicinal application. (3) EriB can improve the levels of reactive oxygen species (ROS), affect IκBα phosphorylation and degradation, and prevent the NF-κB into the nucleus, thereby inhibiting NF-κB signaling pathway. EriB can also downregulate the phosphorylation level of ERK1 / 2, and inhibit MAPKA signaling pathway. (4) EriB can prolong the survival time or reduce tumor size of C57 mice bearing leukemia or in nude mice harboring Kasumi-1 cells, respectively.

### BRIEF DESCRIPTION OF THE DRAWINGS

The foregoing aspects and many of the attendant advantages of this invention will become more readily appreciated as the same becomes better understood by reference to the following detailed description, when taken in conjunction with the accompanying drawings, wherein:
FIG.1A shows the chemical structure of EriB;
FIG.1B shows the IC₅₀ of EriB for some cancer cell lines. For Kasumi-1, NB4, NB4/R1 and NB4/R2 cells, IC₅₀ is less than 1µM;
FIG.1C shows the results of cell growth inhibition and cell survival with various concentrations of EriB in treating Kasumi-1 cells for 24 and 48 hours. The columns in the figure are representatives of mean ± standard deviation of three independent experiments, Kasumi-1 cells treated with 0.25µM EriB are observed with significant cell inhibitory effect and decline in cell survival rate;
FIG.1D shows that Kasumi-1 cells treated with 0.5 or 1µM EriB, the cell shape of NB4 and NB4/R2 cells after 48 hours, which shows a typical apoptosis;
FIG.1E shows Kasumi-1 cells treated with EriB, the Annexin V analysis results of NB4 and NB4/R2 after 24 and 48 hours, EriB increased Annexin-V+/PI- (bottom right) and annexin-V+/PI+ (top right) cell percentage;
FIG. 2A shows Kasumi-1 cell nuclear DNA content distribution after treated with 0.25µM EriB, showing after EriB treatment of Kasumi-1 cells in the sub-G₁ phase cells significantly increased;
FIG. 2B shows ultrastructure of Kasumi-1 cell, and compared to untreated control cells (at the top, 7400 ×), Kasumi-1 cells treated with EriB (below, 7400 ×) showed that mitochondrial damage and the increase in the number of lysosomal (position indicated by arrow) ;
FIG. 2C shows mitochondrial transmembrane potential decline in Kasumi-1 cells treated with EriB for 24 and 48 hours, at bottom of the plot figure represented by Rh123 (Rhodamine) and weak signals of cell proportion;
FIG. 2D shows Caspase-3, active (Δ) caspase-3 and PARP in the western blot analysis, wherein β-actin is used as reference, in Kasumi-1 cells treated with EriB, in NB4 and NB4/R2 cells, caspase-3 is activated, PARP was degraded;
FIG. 3A shows western blot detection of Bcl-2, Bcl-XL and Bax protein expression levels, In Kasumi-1 cells treated with 0.25 and 0.5µM EriB after 24 and 48 hours later, Bcl-2 and Bcl-XL protein levels were downregulated, while Bax was not changed;
FIG. 3B shows using RT-PCR detection of Bcl-2, Bcl-XL and Bax mRNA levels, Kasumi-1 cells treated with 0.25 and 0.5µM EriB after 24 and 48 hours, Bcl-2 and Bcl-XL in the mRNA levels were reduced and Bax did not change;
FIG. 3C shows EriB can reduce Bcl-2/Bax ratio, the bands intensity are quantitatively measured by using densimeter;
FIG. 3D shows EriB can reduce Bcl-X_{L}/Bax ratio, the bands intensity are quantitatively determined by using densimeter;
FIG. 4A shows Luciferase report assay in that EriB downregulate NF-κB -dependent transcription. NF-κB-mediated luciferase report plasmid and β-gal plasmid report instantaneous transfer Kasumi-1 cells. And by 0.25 in 0.5µM EriB treatment for one hour, then add TNF-α incubated for four hours. Columns in the figure are representatives of mean ± standard deviation of three independent experiments (above), and Western blot detection of NF-κB (P65) (below);
FIG. 4B shows EriB inhibit NF-κB DNA-binding capacity. Kasumi-1 cells treatment method: NF-κB-mediated luciferase report plasmid and β-gal plasmid instantaneous transfer Kasumi-1 cells (left), with 0.2mM DTT, 0.25 and 0.5µM EriB and EriB combined with 0.2mM DTT are treated separately for four hours (right). Nucleoprotein extracts were prepared, EMSA experiment was performed to test NF-κB DNA-binding ability;
FIG. 4C shows the immunofluorescence analysis of P65 in Kasumi-1 cells treated with EriB. In untreated Kasumi-1 cells, NF-κB (P65) mainly located in the cytoplasm; treatment with 100ng/ml TNF-α for four hours, NF-κB (P65) was translocated in the nucleus. Pretreatment with EriB for one hour can inhibit TNF-α induced NF-κB (P65) translocation;
FIG. 4D shows that at the presence of TNF-α, EriB can induce IκBα phosphorylation and degradation. P65 protein in nucleus and cytoplasmic were detected by Western blot. Lamin B was used as the control for nuclear protein.
FIG. 4E shows that DTT can inhibit EriB-induced apoptosis and generation of ROS in Kasumi-1 cells. Cells are incubated with 0.2mM DTT and 0.5µM EriB for 12 hours. Mitochondrial transmembrane potential is detected by flow cytometry. Upper panel: DTT decreases Rh123 low staining cells. Cells are treated with 0.2mM DTT and 0.5µM EriB for two hours. Lower panel: DTT reduces increased fluorescent cells. Columns in figure are representatives of mean ± standard deviation of three independent experiments;
FIG. 4F shows EriB-induced IκBα degradation. Kasumi-1 cells are treated with 0.25 and 0.5µM EriB for 24 and 48 hours. Extracted nucleoprotein is used for western blot detection for P65. An anti-carboxyl-terminal (C-IκBα) and an anti-amino-terminal antibody (N-IκBα) for I B are used. IκBα and ΔIκBα bands positions are indicated by arrows;
FIG. 5A shows EriB induced AML1-ETO degradation in Kasumi-1 cells. Cells are treated with 0.25 and 0.5µM EriB for 24 and 48 hours. Whole-cell lysate are detected with an anti-ETO antibody;
FIG. 5B shows Western blot analysis of caspase-3, caspase-3 catabolic fragments (Δcaspase-3), PARP and AML1-ETO. After co-incubation with 40µM Z-DEVD-fmk (caspase-3 inhibitors) for 1 hour, Kasumi-1 cells are treated with 0.5µM EriB for 48 hours. Z-DEVD-fmk can completely inhibit caspase-3 activation, PARP cleavage and AML-ETO degradation;
FIG. 5C shows that Z-DEVD-fmk only partially reduces EriB-induced apoptosis. Kasumi-1 cells are pre-treated with 40µM Z-DEVD-fmk (caspase-3 inhibitors) for 1 hour, followed by co-incubation with 0.5µM EriB for 48 hours. Columns in the figure are representatives of mean ± standard deviation of three independent experiments;
FIG. 6A shows that EriB inhibits ERK phosphorylation. Kasumi-1 cells are treated with EriB, and Western blot is performed to test the expression of phosphorylated p42/44 ERK protein.
FIG. 6B shows that EriB upregulates c-Jun and c-Fos expression.
FIG. 6C shows EriB induced AP-1-dependent transcription activity. Kasumi-1 cells are transfected with AP-1 luciferase report plasmid and β-gal plasmid, followed by treatment with 0.5µM EriB for four hours. Columns in the figure are representatives of mean ± standard deviation of three independent experiments;
FIG. 7A shows that EriB inhibits growth of primary leukemia cells from t(8;21) AML patients. Trypan blue exclusion assay is conducted
FIG. 7B shows that EriB induced apoptosis of primary leukemia cells from t(8;21) AML patients. Morphological examination is performed;
FIG. 7C shows that EriB downregulates Bcl-X_{L} and Bcl-2 expression in primary leukemia cells from t(8;21) AML patients;

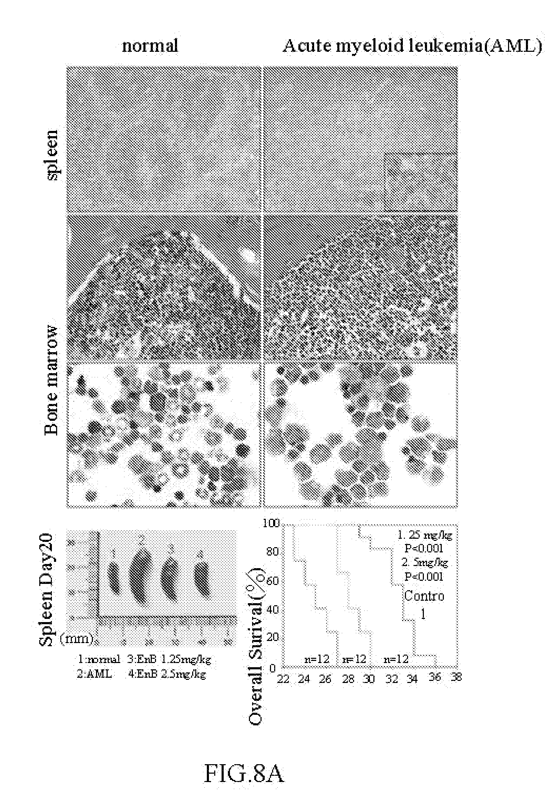

FIG. 8A shows that EriB prolongs the survival time of C57 murine model of t(8;21) leukemia. C57 mice were injected with MigA/Etr cells expressing C-terminal truncated AML1-ETO. Treatments began five days after cell transplantation, continued for two weeks, including solvent control (n = 12), 1.25mg /kg EriB (n = 12) and 2.5 mg / kg EriB (n = 12). Compared with normal C57 mice, mice spleen (100 and 400 times) and bone marrow (400 and 1,000 times) injecting MigA/Etr cells showing leukemia cell infiltration. Compared with the control group (red line: control), EriB can significantly reduce the size of the spleen (20 days), and improve overall total survival time (green Line: 1.25mg/kg EriB, P <0.001; blue Line: 2.5mg/kg EriB, P <0.001 and
FIG. 8B shows that in mice model of tumor xenograft, EriB can reduce tumor size. Subcutaneously inject Kasumi-1 cells to nude mice. Treatment began after 10 days, continued for 10 days, including solvent control (n = 10) and 2.5mg/kg EriB (n = 10). The figure shows the tumors of control mice and EriB treated mice. EriB transplant can significantly reduce the size of xenografted tumors (P <0.05). Every point in the figure represents 10 tumor sizes averaged value ± standard error.

### DETAILED DESCRIPTION OF THE PREFERRED EMBODIMENT

The present invention is described below with the implementation of the preferred embodiments.

Embodiment 1: EriB can inhibit the growth of human leukemia cells and induce apoptosis. Using MTT method to detect the impact of EriB on human malignant hematopoietic cell growth shows that EriB inhibits the growth of these cells at between 0.2 - 2µM, and different concentrations of EriB can also inhibit cell growth. EriB to Kasumi-1 cell growth inhibited half of the growth with inhibitor amount of 0.2µM, to the NB4 and NB4/R2 cells for amount of 0.5µM, and to the NB4R1, HL60 and U937 cells for the amount of 1µM. Lymphocyte proliferation of malignant diseases which are sensitive to EriB are lower than the myeloid leukemia cells, Raji, Daudi, and Jurkat cells and the IC₅₀ values are higher than the 1.5µM (see FIG. 1 B).

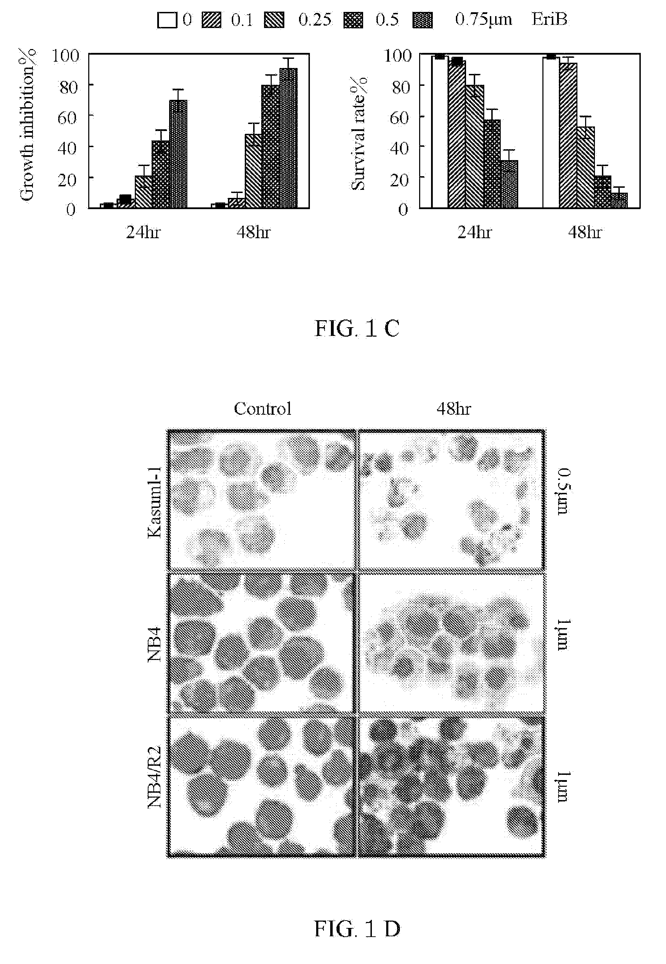

As shown in FIG.1C, Kasumi-1 cells are most sensitive to EriB, EriB to cell growth inhibition has time and dose dependencies (see FIG. 1C right), the survival rate also correspondingly decline (see FIG. 1C right). In less than 0.25µM concentrations, can also observe the inhibitory effect. Kasumi-1 cells treated with 0.25µM and 0.75µM EriB after 48 hours, a total of 42.7 ± 6.5% and 90.5 ± 4.3% cell death (see FIG. 1C right).

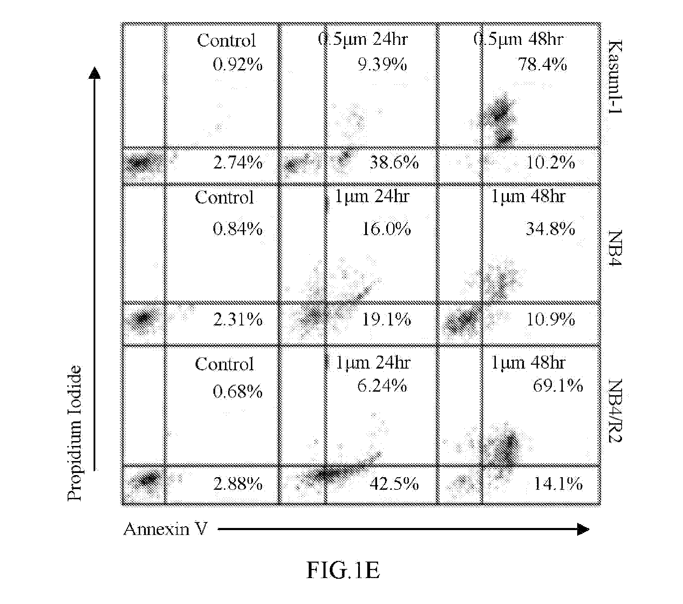

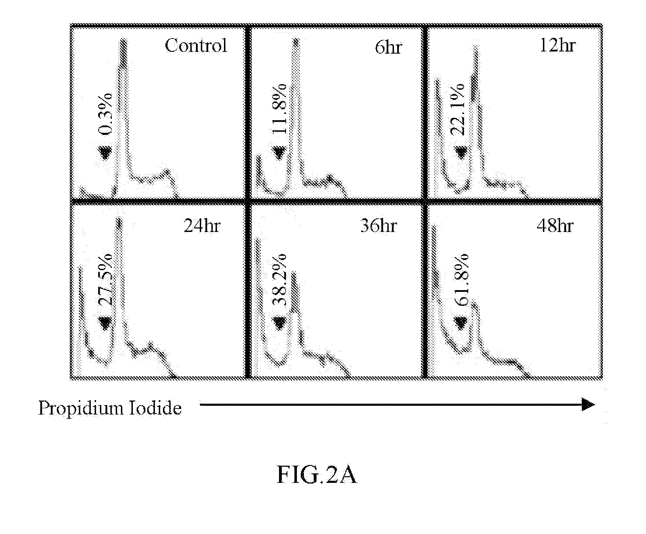

In order to detect whether EriB through induction of apoptosis leads to cell growth inhibition, detections of Kasumi-1 cells form and annexin V-FITC/PI (propidium iodide) were performed. The Kasumi-1 cells treated with 0.5µM EriB showed the characteristics of apoptosis morphological changes, for example: shrinkage of the cytoplasm, condensed chromatin, and nuclear fragmentation with intact cell membrane, the NB4 and NB4/R2 cells treated with 1µM EriB also showed similar features (see FIG. 1D). Kasumi-1 cells treated with 0.5µM EriB after 48 hours, annexin V-positive cells proportion increased to 88.6%, this ratio is higher than the NB4 and NB4/R2 cells treated with 1µM EriB for 48 hours (see FIG. 1E). Kasumi-1 cells treated with 0.25µM EriB for 6, 12, 24, and 48 hours, in the cell cycle analysis showed higher proportion of sub-G1 indicating EriB-induced apoptosis, but no changes in the cell cycle (see fig. 2A).

Kasumi-1 cells treated with 0.1 and 0.25µM EriB for seven days have not changed their myeloid cells and monocytes differentiation associated antigen expression, such as: CD11b, CD13, CD14, CD33, CD34 and CD64; and did not induce differentiation.

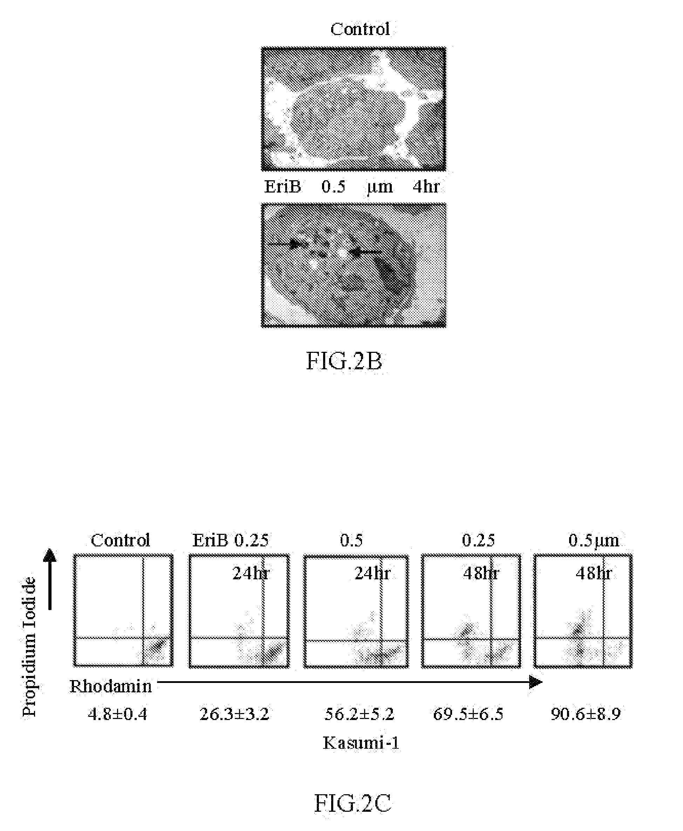

Embodiment 2: Mitochondrial damage by EriB in induced apoptosis concentration, activated caspase-3. In order to study the Kasumi-1 cells in early ultrastructural changes of apoptosis and to find the mechanism involved in apoptosis, we had a transmission microscope analysis. The normal tumor cells nucleus are irregularly shape, containing a lot of rough endoplasmic reticulum and mitochondria (see FIG. 2B), Kasumi-1 cells treated with EriB for six hours later showed expansion of the cell, the increase in lysosome number, swelling and debilitated mitochondria (see FIG. 2B), which prompted the early cell apoptosis and mitochondrial damage (see FIG. 2B).

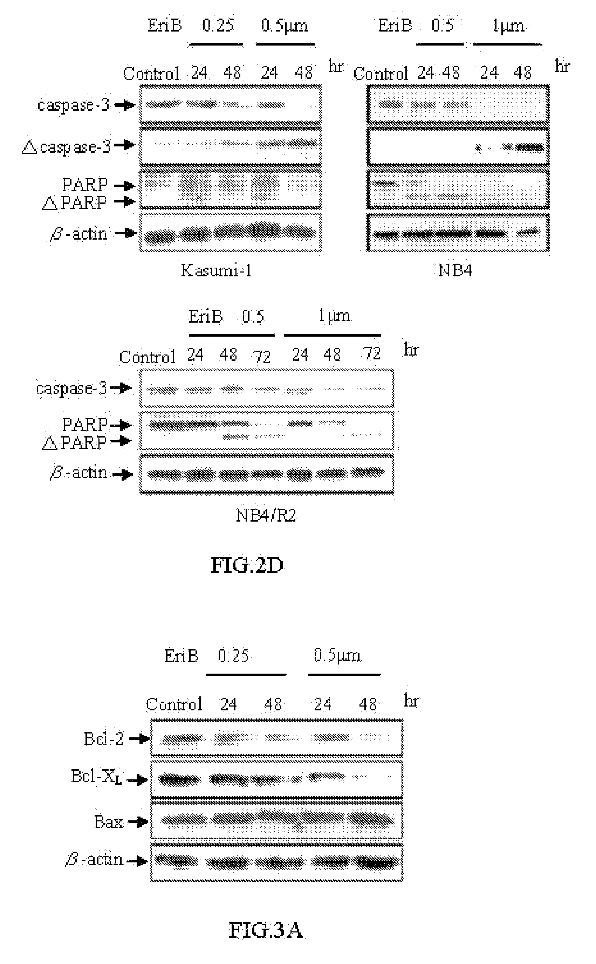

The Kasumi-1 cells treated with EriB and Rh123 (Rhodamine) after incubation are used for the flow cytometry detection, the results showed that mitochondrial transmembrane potentials decreased significantly, and have time and dose dependencies (see FIG. 2C). NB4 and NB4/R2 cells in Kasumi-1 cells, caspase-3 cleaving also occurred simultaneously with the mitochondrial transmembrane potential changes (see FIG. 2D). PARP is a ribozyme involved in DNA repair, when DNA received injuries, it will be cleaved specifically into 85-kDa fragments (ΔPARP), in EriB treatment process, PARP was also degraded (See FIG. 2D). These results suggest that EriB through dependency on the activation of caspase intrinsic mitochondrial pathways to induce apoptosis.

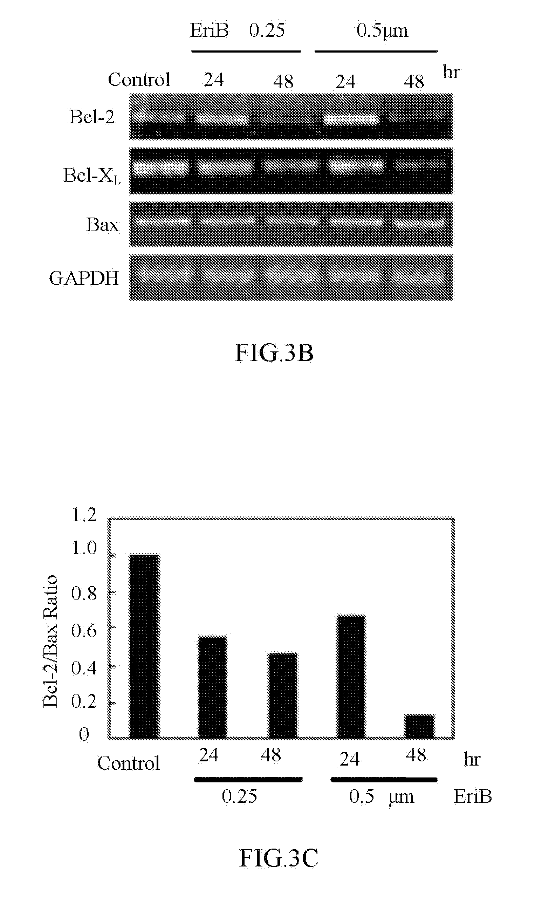

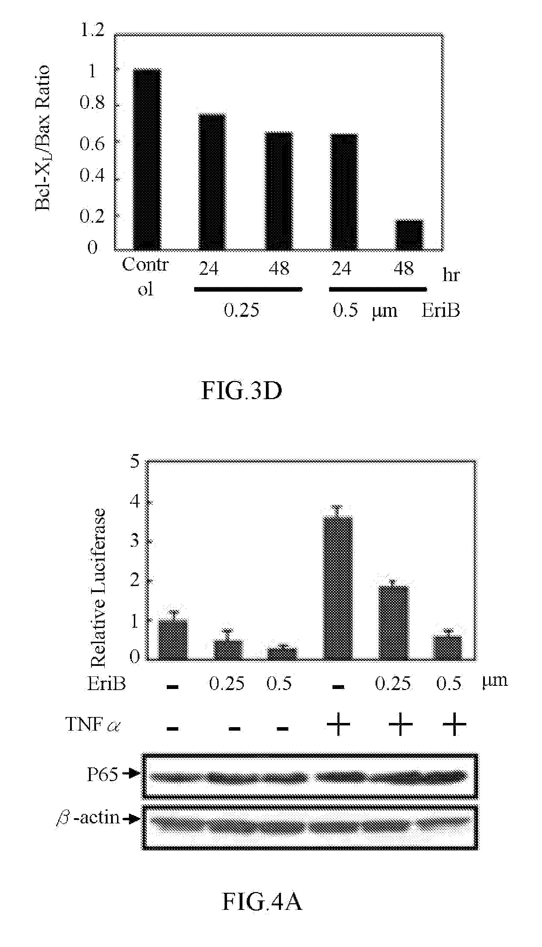

Embodiment 3: EriB through downregulation of Bcl-2 and Bcl-X_{L} to act on intrinsic apoptosis pathways. Bcl-2 family proteins directly control the mitochondrial membrane permeability, and they are caspase activation central regulators. The family of anti-apoptotic and pro-apoptotic members decided the fate of the cells whether to live or die. In order to detect whether EriB through the role of EriB in the Bcl-2 family members to act on mitochondrial damage, we used western blot and RT-PCR methods to detect the treatment of EriB for 24 and 48 hours later, Kasumi-1 cells situations in the anti-apoptotic factor Bcl-2 and Bcl-X_{L,} and pro-apoptotic factor Bax expression. Bcl-2 and Bcl-X_{L} in protein and mRNA levels have decreased, but the expressions of Bax have not changed significantly (see FIG. 3A and FIG. 3B). Ultimately, EriB induced Bcl-X_{L}/Bax Bcl-2/Bax ratio and the negative regulated (see FIG. 3C and FIG. 3D), and the results were consistent with Kasumi-1 apoptosis process of mitochondrial stability damage.

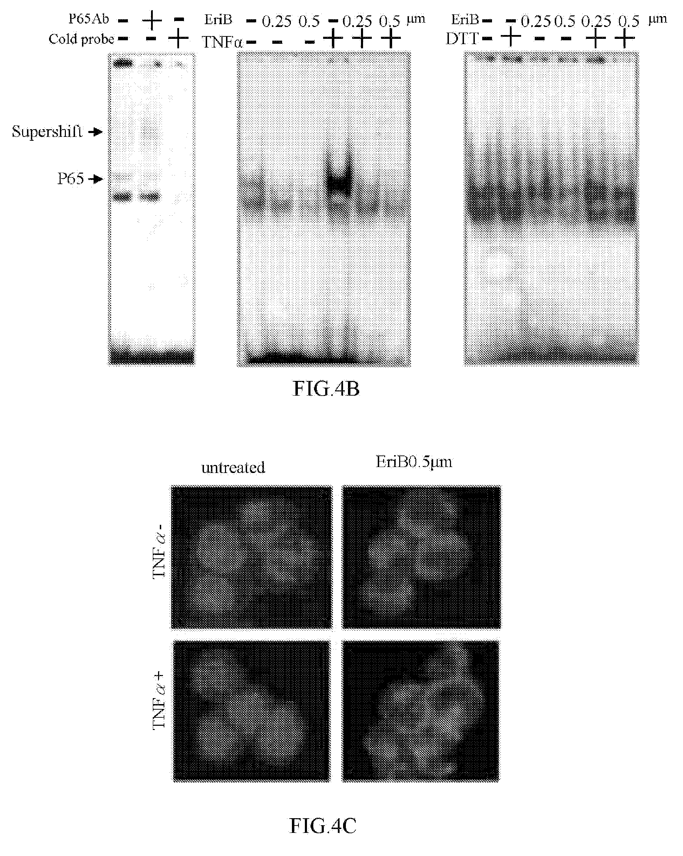

Embodiment 4: EriB inhibited intrinsic and TNF-α-induced NF-κB activation of Kasumi-1 cells. EriB can reduce Bcl-2 and Bcl-X_{L} at the mRNA level, and they are directly affecting by the transcription of NF-κB control. In order to study whether EriB can block intrinsic-mediated and TNF-α-mediated NF-κB activation, we proceeded with luciferase report assay. In Kasumi-1 cells, the NF-κB showed that the basis of the transcription activity, which prompted that NF-κB is constitutively activated, joining with the TNF-α 4 hours later, the luciferase activity approximately increased four times (see FIG. 4A ). Pre-treatment of cells with EriB for one hour, base luciferase activity and TNF-α-induced NF-κB activities have been blocked (see FIG. 4A). However, EriB and TNF-α individually or jointly have not affected the P65 protein levels (see FIG. 4A). EMSA experiments showed Kasumi-1 cells after treated with the TNF-α displayed significantly increased activity of NF-κB (see FIG. 4B), where as EriB added one hour before treatment with EriB can block NF-κB activation (see FIG. 4B). However, the inhibitory effect of formation of disulfide compounds DTT can reduce the EriB in the inhibitory effect (see FIG. 4B). This suggested that the inhibitory effect of EriB is redox sensitive. We also analyzed the Kasumi-1 cells specific in the NF-κB complex, slower migration protein - DNA complex corresponding to P65-P50 dimers. These results suggest that NF-κB in Kasumi-1 cells is constitutively activated, in the EMSA experiment, EriB can inhibit intrinsic-induced and TNF-α-induced NF-κB activities.

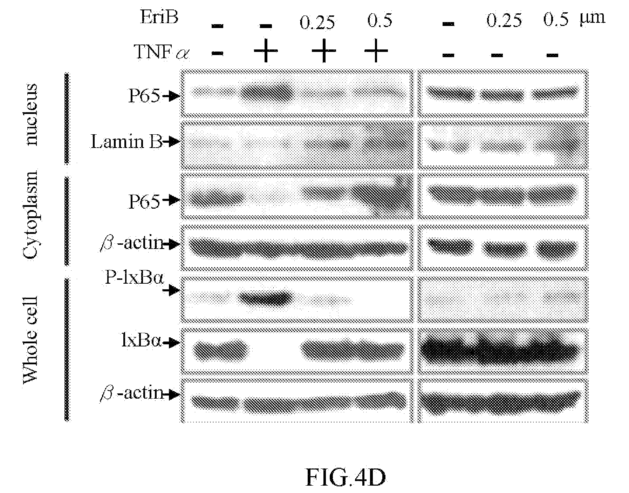

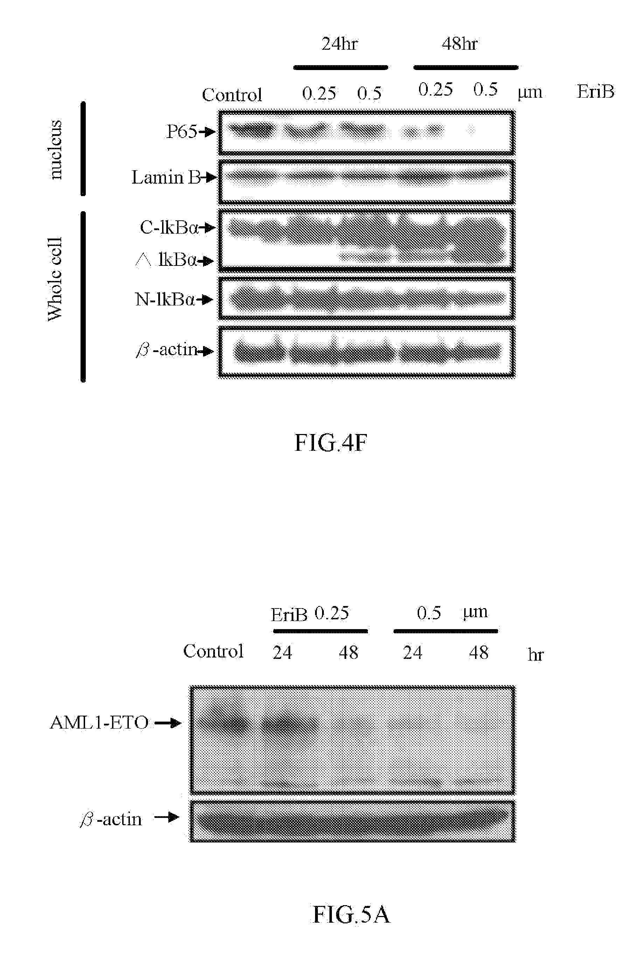

Embodiment 5: EriB prevented NF-κB nuclear translocation and IκBα degradation in Kasumi-1 cells. In order to further understand EriB-mediated NF-κB deactivation mechanism, we prepared immunofluorescence detection and western blot analysis. Despite that the Kasumi-1 cells' EriB had no apparent impact on nuclear translocation of P65, it can significantly prevent TNF-α-induced P65 from rapidly translocate into nucleus (see FIG. 4C). Wherein Kasumi-1 cells treated with EriB, the nuclear NF-κB is reduced, and increased in the cytoplasm (see FIG. 4D). In order to detect EriB effect to nucleus NF-κB, we prepared Kasumi-1 cell nuclear protein extract. After treated with EriB for 24 and 48 hours, Kasumi-1 cells nuclear protein is reduced (see FIG. 4F).

IκBα regulates NF-κB activities by isolating NF-κB in the cytoplasm. IκBα, after the ubiquitination and proteasomal degradation, will lead to NF-κB nuclear translocation. In the present invention studies, TNF-α induced IκBα phosphorylation in four hours, but the protein phosphorylation in pretreatment of EriB was inhibited (see FIG. 4D). In order to detect the role of IκBα in the process of reducing the nucleus level of NF-κB, we used Western blot analysis on overall protein of Kasumi-1 cells treated with EriB. We used anti-IκBα -carboxyl -terminal antibody and detected two protein forms, 37kDa and 34kDa; but with anti-IκBα-amino-terminal antibody, we can not detect protein forms 34kDa (see FIG. 4F). These results suggest that EriB induce IκBα to be cleaved into protein forms the size of 34kDa (ΔIκBα). ΔIκBα lacks of ubiquitination sites, through the NF-κB stability isolated in the cytosol to produce the effect of super-inhibitor.

In summary, EriB can inhibit the intrinsic NF-κB activity by directly inhibit NF-κB and DNA binding ability, and mainly through the inhibiting IκBα phosphorylation and degradation to block TNF-α-induced NF-κB activity.

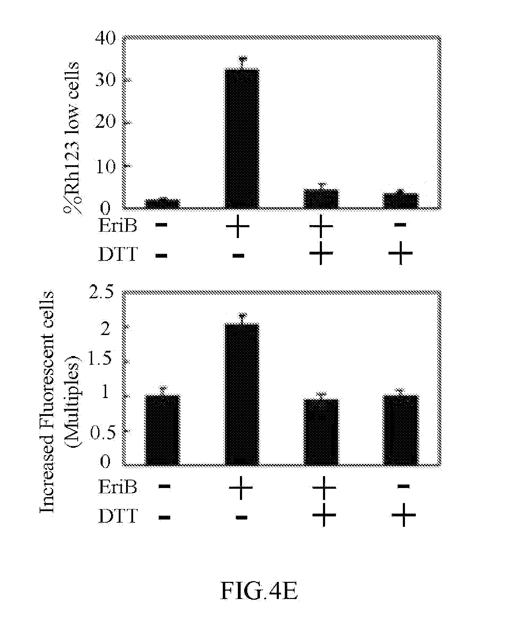

Embodiment 6: DTT inhibited EriB induced Kasumi-1 apoptosis and increased levels of reactive oxygen species (ROS). Some researches show that EriB through interaction with activated SH-group to have toxic effects. In order to prove whether this mechanism is involved in the EriB induced apoptosis, we used 0.5µM EriB and 0.2mM DTT to continuously treat Kasumi-1 cells. Cells independently treated with DTT to Rh123 cells proportion have no impact (see FIG. 4E). However, 0.2mM DTT can completely inhibit EriB-induced apoptosis (see FIG. 4E). In order to study whether ROS is related to EriB-induced apoptosis, we used a cell permeability dye H2DCFDA to proceed with flow cytometry analysis. In the presence of ROS, H2DCFDA be specifically cleaved and emit a certain wavelength of fluorescence. Kasumi-1 cells independently treated with 0.5µM EriB for 2 hours, the cell level of ROS compared with untreated cells increased about two times, but when the 0.2mM DTT and EriB jointly incubated, ROS level has not changed (see FIG. 4E).

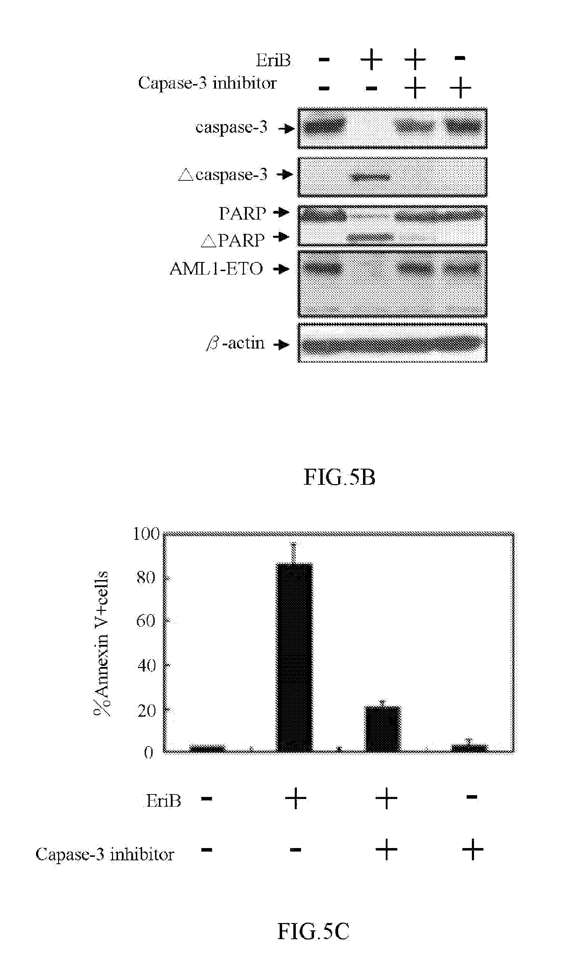

Embodiment 7: EriB induced degradation of AML-ETO fusion protein. This change can antagonize caspase-3 inhibitors. AML-ETO fusion protein is generated by the chromosome translocation of t(8;21), AML-ETO is considered an important therapeutic target into the treatment of AML comprising fusion protein. We used anti-ETO antibody testing found that Kasumi-1 cells after treated with EriB for 24 and 48 hours AML1-ETO degradation (see FIG. 5A), which indicated EriB directly to fusion protein. AML1-ETO degradation and caspase-3 activation occurred in the same time, therefore we studied further the process of EriB in the induction of apoptosis, caspase-3 activation and AML1-ETO degradation relationship. In the process of Kasumi-1 cells treated with EriB, pre-treatment with the caspase-3 inhibitor Z-DEVD-fmk (40µM). Z-DEVD-fmk can completely block EriB induced caspase-3 activity, PARP degradation and AML-ETO degradation (see FIG. 5B). However, in the Kasumi-1 cells treated with 0.5µM EriB, pre-treatment of Z-DEVD-fmk and incubated for one hour, the rate of apoptosis (annexin V positive cell proportion) only partially reduced (see FIG. 5C). These results suggest that EriB induced AML1-ETO-degradation is accompanied by caspase-3 incident. However, in EriB induced apoptosis, in addition to activation of caspase-3, there is still a non-dependent caspase-3 mechanism.

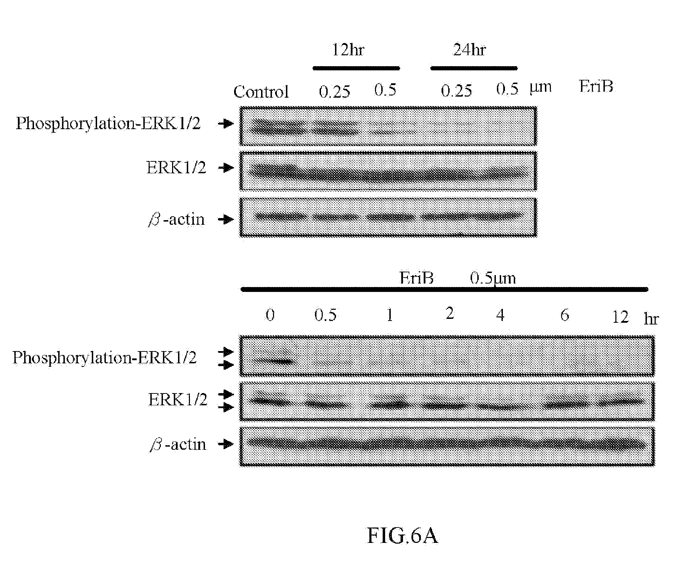

Embodiment 8: EriB inhibit ERK pathway and initiate AP-1 pathway in Kasumi-1 cells. The Ras / Raf / MEK / ERK pathway is an important signaling cascade response involved in controlling the proliferation of blood cells, the destruction of this pathway will predominate pro-apoptotic signals in AML. In order to detect ERK activation in response to EriB, we used anti-ERK1 / 2 phosphorylation forms (p-ERK1 / 2) antibody to proceed with western blot analysis. In Kasumi-1 cells, EriB treated for 12 and 24 hours later, ERK1 / 2 phosphorylation level rapidly decreased (see FIG. 6A). In fact, Kasumi-1 cells treated with EriB for 30 minutes, p-ERK1 / 2 has already begun to reduce (see FIG. 6A).

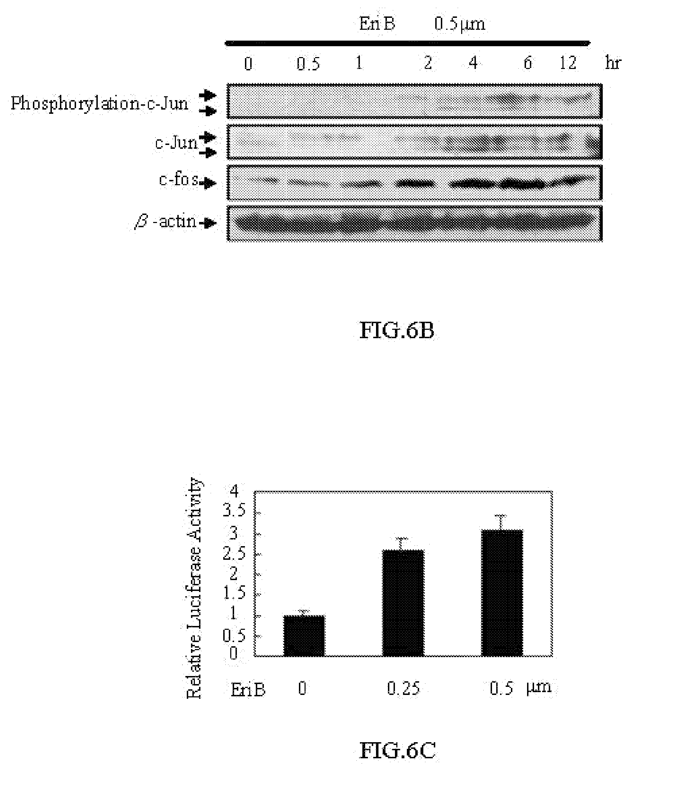

Early in vitro experiments show that increased AP-1 activation can lead to specific human tumor cell apoptosis. Mammals AP-1 protein complexes are divided into main groups of c-Jun and c-Fos. In Kasumi-1 cells, EriB can quickly induced c-Jun, c-Fos and phosphorylation of c-Jun expression (see FIG. 6B). C-Jun phosphorylation forms increased means c-Jun activation indicated that the transcription factor-induced apoptosis in EriB play a potential role. In order to further assess EriB effect on AP-1 activity, we proceeded with luciferase report assay. In Kasumi-1 cells instantaneous transfer of pAP-1-Luc plasmid report, adding EriB for 4 hours after, the luciferase activity increased about three times (see FIG. 6C), which prompted EriB in Kasumi-1 cells by inducing c-Jun, c-Fos and c-Jun phosphorylation forms of expression to enhance the transcription factor of AP-1 activity.

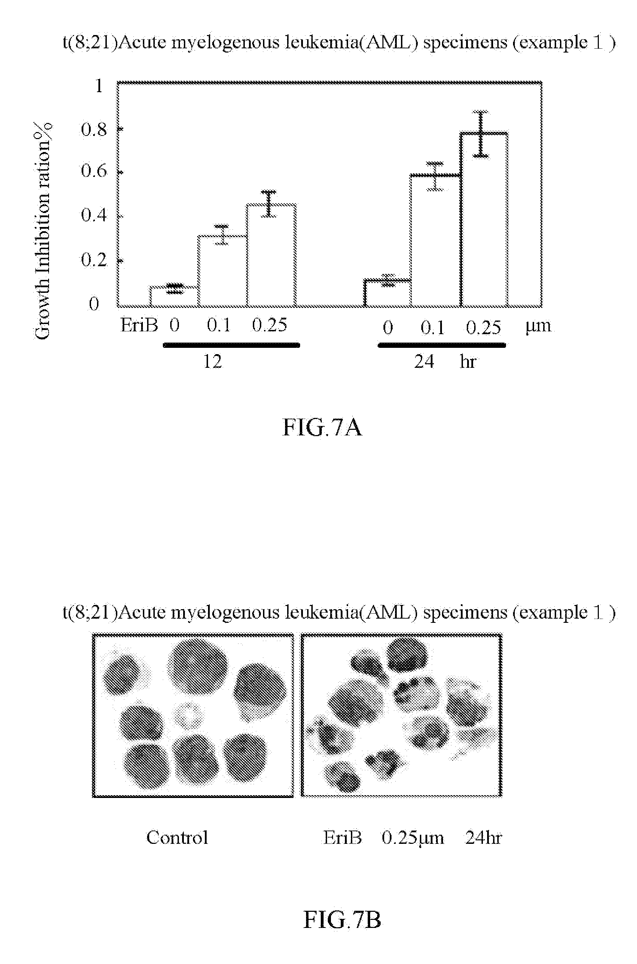

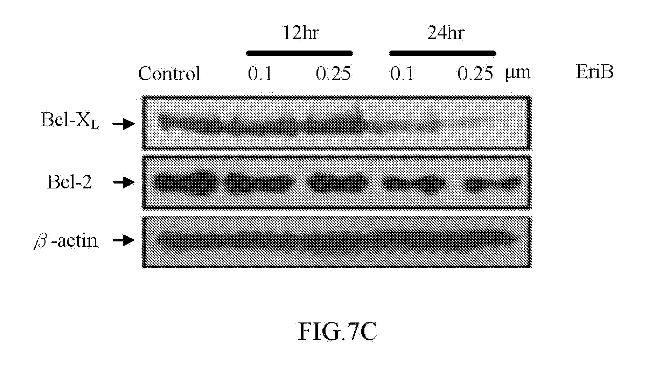

Embodiment 9: EriB induced apoptosis of leukemia from patients with t(8;21). Treatment with 0.1 and 0.25µM EriB in primary leukemia cells from patients with t(8;21), can significantly inhibit cell growth in all five cases of patients reached more than 60% of the cell inhibition (see FIG. 7A). Treatment with 0.1µM EriB for 24 hours, we observed that the typical characteristics of apoptosis with morphological changes (see FIG. 7B). EriB in treatment after 12 and 24 hours, can also lower Bcl-2 and Bcl-X_{L} level (see FIG. 7C). These results suggest that the primary leukemia cells are very sensitive to EriB.

Embodiment 10: EriB efficacy in the treatment of murine models. C57 mice were irradiated by lethal dose (LD)₅₀ and injected via tail vein intravenously with 3×10⁶ MigA/Etr cells expressing of C-terminal truncated AML1-ETO (day 0). Beginning on day 5, intraperitoneal injection of EriB (1.25 or 5mg/kg) for two weeks. The overall survival rate was measured and calculated started from day 0 to the date of death. All the mice eventually develop symptoms of anemia and difficulty breathing, and also the occurrence of a large number of primitive blood cells in the peripheral blood. Histological and morphological analyses show that compared to the normal C57 mice, the incidence mice hematopoietic organs (spleen and bone marrow) structures are destroyed and a large number of immature parental cells are invaded (see FIG. 8A). The mice treated with EriB had spleen size obviously smaller than untreated mice (see FIG. 8A). Moreover, in the animal models, EriB can significantly delay disease onset, increase survival time in mice, and extend the median survival time from 25 days (control group) to 28 days (EriB 1.25 mg/kg, P<0.001) or 32 days (EriB 2.5 mg/kg, P<0.001) (see FIG. 8A).

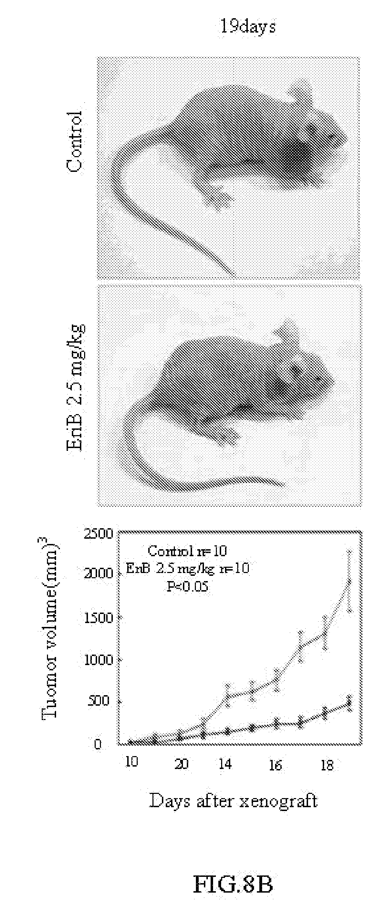

Another xenograft tumor model was established through subcutaneous injection in nude mice of 3×10⁷ Kasumi-1 cells (day 0). The incubation period of tumor formation at the site of injection was about 8-12 days. Beginning from day 10, the mice were treated with EriB (2.5 mg/kg) by intraperitoneal injection only until day 19. Compared with the control group, EriB can significantly reduce tumor size (P <0.05) (see FIG. 8B).

It is to be understood, however, that even though numerous characteristics and advantages of the present invention have been set forth in the foregoing description, together with details of the method and function of the invention, the disclosure is illustrative only, various modifications and changes may be made by persons skilled in this art, especially in arrangement of parts within the principles of the invention to the full extent indicated by the broad general meaning of the terms in which the appended claims are expressed. It is intended that the present invention should not be limited to the particular forms, and that all modifications and changes which maintain the spirit and realm of the present invention are within the scope as defined in the appended claims.

## Claims

1. An application of eriocalyxin B in manufacturing medicaments for treating leukemia.

2. The application according to claim 1, wherein eriocalyxin B is used in manufacturing medicaments for treating leukemia with AML1-ETO fusion gene.

3. The application according to claim 1, wherein eriocalyxin B is used in manufacturing medicaments for treating acute promyelocytic leukemia.

4. The application according to claim 1, wherein eriocalyxin B is used in manufacturing medicaments for treating acute myelogenous leukemia.

5. The application according to claim 1, wherein eriocalyxin B is used in manufacturing medicaments for treating lymphocytic leukemia.

6. An application of eriocalyxin B in manufacturing medicaments for treating other malignant tumors.
